# FASCICULE DE BREVET EUROPEEN

(11) **EP 3 449 256 B1**
(45) Date de publication et mention de la délivrance du brevet: **03.09.2025**
(21) Numéro de dépôt: 17719243.2
(22) Date de dépôt: 24.04.2017
(51) Int. Cl.: G01N 33/564, G01N 33/68, C07K 16/06, C07K 16/24, A61K 39/395, A61P 19/00, A61P 37/00

(54) **ANTICORPS ANTI IL17**
ANTI-IL17 ANTIKÖRPER
ANTI-IL17 ANTIBODY

(30) Priorité: 25.04.2016 FR 1653646
(43) Date de publication de la demande: 06.03.2019
(73) Titulaire: Hospices Civils De Lyon, 69002 Lyon (FR); Université Claude Bernard Lyon 1, 69100 Villeurbanne (FR); Dendritics, 69008 Lyon (FR)
(72) Inventeur: MIOSSEC, Pierre, 69500 BRON (FR); THIAM, Ndiémé, 38640 CLAIX (FR); PIN, Jean-Jacques, 69720 SAINT BONNET DE MURE (FR)
(74) Mandataire: Ipsilon
(86) Numéro de dépôt international: PCT/EP2017/059638
(87) Numéro de publication internationale: WO 2017/186631

(56) Documents cités:
- WO-A1-2015/186106
- US-A1- 2014 363 422
- GENOVESE M.C. ET AL.: "A phase II randomized study of subcutaneous Ixekizumab, an anti-interleukin-17 monoclonal antibody, in rheumatoid arthritis patients who were naive to biologic agents or had an inadequate response to tumor necrosis factor inhibitors", ARTHRIT. RHEUMATOL., vol. 66, no. 7, July 2014 (2014-07-01), pages 1693 - 1704, XP055303046
- HUEBER W. ET AL.: "Effects of AIN457, a fully human antibody to interleukin-17A, on psoriasis, rheumatoid arthritis, and uveitis", SCI. TRANSL. MED., vol. 2, no. 52, 52RA72, October 2010 (2010-10-01), pages 1 - 9, XP009155660
- JOUVENNE P. ET AL.: "High levels of neutralizing autoantibodies against IL-1 alpha are associated with a better prognosis in chronic polyarthritis: a follow-up study", SCAND. J. IMMUNOL., vol. 46, no. 4, October 1997 (1997-10-01), pages 413 - 418, XP002410039
- MIOSSEC P.: "Anti-interleukin 1alpha autoantibodies", ANN. RHEUM. DIS., vol. 61, no. 7, July 2002 (2002-07-01), pages 577 - 579, XP002668460
- NDONGO-THIAM N. ET AL.: "A cell-based bioassay for circulating bioactive IL-17: application to destruction in rheumatoid arthritis", ANN. RHEUM. DIS., vol. 74, no. 8, August 2015 (2015-08-01), pages 1629 - 1631, XP008177077
- NDONGO-THIAM N. ET AL.: "Negative association between autoantibodies against IL-17, IL-17/anti-IL-17 antibody immune complexes and destruction in rheumatoid arthritis", ANN. RHEUM. DIS., vol. 75, no. 7, 29 April 2016 (2016-04-29), pages 1420 - 1422, XP009191683

## Description

### DOMAINE DE L'INVENTION

La présente invention est relative à l'identification de marqueurs biologiques de la polyarthrite rhumatoïde (PR). L'invention décrit un procédé de détermination du niveau de ces nouveaux biomarqueurs, de nouveaux anticorps ainsi que leurs applications thérapeutiques, diagnostiques ou pronostiques.

### ART ANTERIEUR

Les maladies auto-immunes sont généralement caractérisées par le déclenchement d'une réaction immunitaire, qui se traduit par une réaction inflammatoire, à l'égard de substances et/ou tissus normalement présents chez l'individu. Elles sont souvent mises en évidence, mais pas systématiquement, par la détection d'auto-anticorps dirigés contre des antigènes du « soi ».

Ces maladies auto-immunes et inflammatoires chroniques sont des pathologies hétérogènes, pour lesquelles il existe une forte variabilité des phénotypes et des réponses aux traitements. Ainsi, le pronostic de ces maladies auto-immunes et inflammatoires chroniques est susceptible de varier considérablement au sein d'individus pourtant considérés comme atteints de la même pathologie.

En particulier, la polyarthrite rhumatoïde est une maladie dégénérative inflammatoire chronique, caractérisée par une atteinte articulaire souvent bilatérale et symétrique, évoluant par poussées vers la déformation et la destruction des articulations atteintes. Le traitement symptomatique fait appel à des anti-inflammatoires non-stéroïdiens et à des corticostéroïdes. De nos jours, le méthotrexate est le traitement de référence. Des traitements « de fond » de la maladie sont activement recherchés, notamment par identification des cytokines et des cellules impliquées dans le processus inflammatoire de la maladie. Ces traitements font appel à des inhibiteurs spécifiques des cytokines et des cellules impliquées. On peut citer notamment l'Etanercept^{®} et l'Infliximab^{®}, inhibiteurs du TNF (Tumor Necrosis Factor). D'autres traitements mettent en œuvre des antagonistes d'interleukines tels que des inhibiteurs de l'IL-1 (Anakinra^{®}), du récepteur de l'IL-6 (MrA^{®}) et les anti-CD20 (Rituximab^{®}). Ces composés réduisent l'inflammation, et ralentissent l'évolution de la maladie.

Néanmoins, une forte variation de réponse est observée entre les patients atteints de polyarthrite rhumatoïde, qui ne répondent pas de manière similaire à ces traitements. On estime notamment que jusqu'à 30 % des patients ne répondent pas du tout aux biothérapies listées ci-dessus.

Jouvenne P. et al., Scand. J. Immunol., 1997, 46(4): 413-418 rapporte une étude de suivi de l'évolution de patients souffrants de PR avec et sans destruction osseuse qui montre que des niveaux élevés d'anticorps neutralisants dirigés contre I'IL-1α sont associés à un meilleur pronostic de la PR et conclut que les auto-anticorps naturels contre I'IL-1α peuvent protéger contre ou retarder l'apparition de la PR avec destruction osseuse et agir d'une manière dose-dépendante pour l'évolution de la maladie.

Il a été montré que l'IL-17 joue un rôle majeur dans les maladies inflammatoires, les maladies auto-immunes, certaines infections et le cancer. Ainsi, cette cytokine est maintenant considérée comme une cible thérapeutique potentielle pour de nombreuses maladies (Miossec et al., N. Engl. J. Med. 2009, 361(9): 888-898). Les deux formes majoritaires de cette cytokine sont dénommées IL-17A et IL-17F. L'IL-17A induit la production de nombreuses cytokines et chémokines, telles que l'IL-6, le G-CSF, I'IL-1β, et l'IL-8. Dans la polyarthrite rhumatoïde, l'IL-17 est présente aux sites d'inflammation et agit en amplifiant les effets inflammatoires d'autres composés tels que le TNF, ce qui en fait un acteur important dans la physiologie de la maladie. Cependant, les taux circulants d'IL-17 dans le plasma des patients sont souvent très faibles, et une très grande hétérogénéité des taux circulants d'IL-17 est observée entre les patients.

Des inhibiteurs de l'IL-17A, notamment des anticorps, ont été proposés pour le traitement de maladies inflammatoires, et différents essais cliniques sont en cours (Miossec et Kolls, Nat. Rev. Drug Discov. 2012, 11(10): 763-776). Les premiers résultats obtenus indiquent qu'il est difficile de prédire quelle sera la réponse des patients à ces traitements, car une très forte variation individuelle existe.

Chez certains patients, le rôle de l'IL-17 dans le phénomène inflammatoire est important, alors que chez d'autres patients celui-ci est très faible. Un effort majeur doit être maintenant porté à la pré-sélection des patients dont l'état inflammatoire est directement dépendant de l'IL-17, car ce sont eux qui seraient bénéficiaires de ce type de traitement.

WO2015/186106 enseigne notamment des procédés *in vitro* mettant en œuvre le niveau d'IL-17 fonctionnelle (IPDL) en tant que biomarqueur, pour déterminer les chances de réponse d'un patient atteint d'un état inflammatoire chronique à l'administration d'un anticorps anti-IL17.

Ainsi, il demeure un besoin d'identifier de nouveaux marqueurs de la polyarthrite rhumatoïde. Il demeure également un besoin d'identifier de nouveaux procédés et outils pour le diagnostic, pronostic et/ou traitement de cette maladie auto-immune et inflammatoire chronique.

### RESUME DE L'INVENTION

La présente invention décrit un biomarqueur de la polyarthrite rhumatoïde, basé sur la détection dans un échantillon biologique d'un auto-anticorps anti-IL-17, lequel peut être déterminé soit au regard d'un niveau total, soit en complexe avec l'Interleukine-17.

La présente invention concerne donc un procédé *in vitro* pour évaluer le pronostic de la polyarthrite rhumatoïde chez un individu, comprenant les étapes suivantes :
**a)** déterminer (i) le niveau d'un auto-anticorps anti-IL-17 et/ou (ii) le niveau d'un complexe [IL-17/auto-anticorps anti-IL-17] dans un échantillon biologique dudit individu, et
**b)** comparer le niveau d'auto-anticorps et/ou de complexe déterminé à l'étape a) avec une valeur de référence, la dite comparaison étant indicative du pronostic de la polyarthrite rhumatoïde chez le dit individu.

Les précédents procédés permettent aussi d'identifier des populations particulières d'individus, telles que des populations particulières d'individus atteints de polyarthrite rhumatoïde, pour lesquelles un principe actif inhibiteur de l'IL-17 est administrable.

La connaissance de ce nouveau biomarqueur permet notamment les applications diagnostiques et/ou pronostiques suivantes :
(i) déterminer le risque de sévérité, en particulier de destruction osseuse, chez un individu affecté d'une polyarthrite rhumatoïde ;
(ii) déterminer les chances de réponse d'un individu atteint d'une polyarthrite rhumatoïde, à un traitement comprenant l'administration d'un principe actif inhibiteur de l'IL-17, tel qu'un anticorps anti-IL17.
(iii) déterminer l'efficacité d'un traitement ou d'une prévention d'une destruction osseuse d'un individu atteint d'une polyarthrite rhumatoïde, ledit traitement ou la dite prévention consistant en l'administration d'un principe actif inhibiteur de l'IL-17, tel qu'un anticorps anti-IL-17.

En particulier, la présente invention décrit un procédé *in vitro* de sélection d'anticorps pour traiter (i) une polyarthrite rhumatoïde, et/ou (ii) une destruction osseuse associée à la dite maladie, comprenant les étapes suivantes :
**a)** déterminer la présence (i) d'un auto-anticorps anti-IL-17 et/ou (ii) d'un complexe [IL-17/auto-anticorps anti-IL-17] dans un échantillon biologique d'un individu évalué par ledit procédé *in vitro* ou selon la revendication 1 ;
**b)** sélectionner un auto-anticorps anti-IL-17 ou une cellule productrice d'un auto-anticorps anti-IL-17 à partir d'un échantillon biologique du dit individu ; le dit auto-anticorps étant apte à traiter la dite maladie, et/ou la dite destruction osseuse.

La présente invention concerne également un procédé *in vitro* pour distinguer une polyarthrite rhumatoïde destructrice d'une polyarthrite rhumatoïde non destructrice chez un individu, comprenant les étapes suivantes :
**a)** déterminer (i) le niveau d'un auto-anticorps anti-IL-17 et/ou (ii) le niveau d'un complexe [IL-17/auto-anticorps anti-IL-17] dans un échantillon biologique dudit individu, et
**b)** comparer le niveau d'auto-anticorps et/ou de complexe déterminé à l'étape a) avec une valeur de référence, la dite comparaison étant indicative d'une polyarthrite rhumatoïde destructrice ou non destructrice chez le dit individu.

La présente invention concerne également un anticorps anti-IL-17 isolé pour le traitement ou la prévention (i) d'une polyarthrite rhumatoïde destructrice et/ou (ii) d'une polyarthrite rhumatoïde chez un individu ne produisant pas d'auto-anticorps anti-IL-17, ou d'une destruction osseuse associée à l'un quelconque des groupes (i) ou (ii).

La présente invention concerne également l'utilisation d'un anticorps anti-IL-17 isolé pour la préparation d'un médicament pour le traitement ou la prévention (i) d'une polyarthrite rhumatoïde destructrice et/ou (ii) d'une polyarthrite rhumatoïde chez un individu ne produisant pas des auto-anticorps anti-IL-17, ou d'une destruction osseuse associée à l'un quelconque des groupes (i) ou (ii).

### LEGENDE DES FIGURES

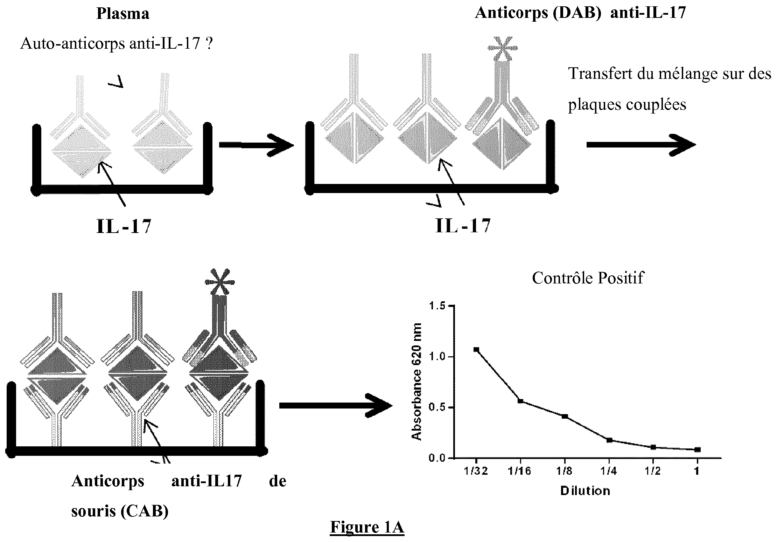

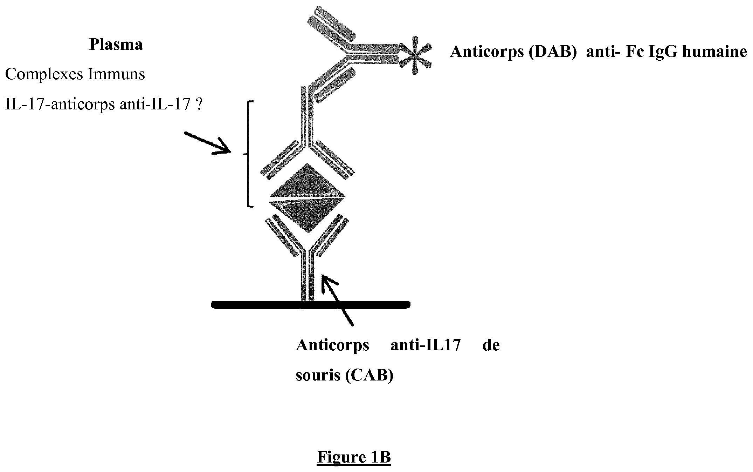

**Figure 1** **: Principe de détection d'auto-anticorps anti-IL-17 et de complexes IL-17/anticorps anti-IL-17.**
   Les autoanticorps anti-IL-17 sont détectés dans le plasma par un test ELISA de compétition (A). L'IL-17 et le plasma sont combinés pour promouvoir la fixation de l'IL-17 et de l'anticorps anti-IL-17. L'anti-IL-17 et des anticorps non pertinents ont été utilisés respectivement comme contrôles positifs et négatifs. Un anticorps anti-IL17 de détection a été ensuite ajouté. Le mélange est ensuite transféré vers des plaques comprenant l'anticorps de capture anti-IL-17 humaine.
   Les complexes [IL-17/auto-anticorps anti-IL-17] sont détectés dans le plasma par un test ELISA indirect **(B).** Dans une plaque 96 puits comprenant l'anticorps de capture anti-IL-17, du plasma pré-incubé avec du sérum de cheval est rajouté. Le complexe immun (CI) contrôle positif a été formé *in vitro* à partir de 500 ng de IL-17 et 5 µg/ml d'anticorps humain anti-IL1-17 humaine. Un anticorps de chèvre anti-Fragment Fc d'IgG humaine (anti-IgG Fc humaine) conjugué à la peroxydase a été utilisé pour la révélation.

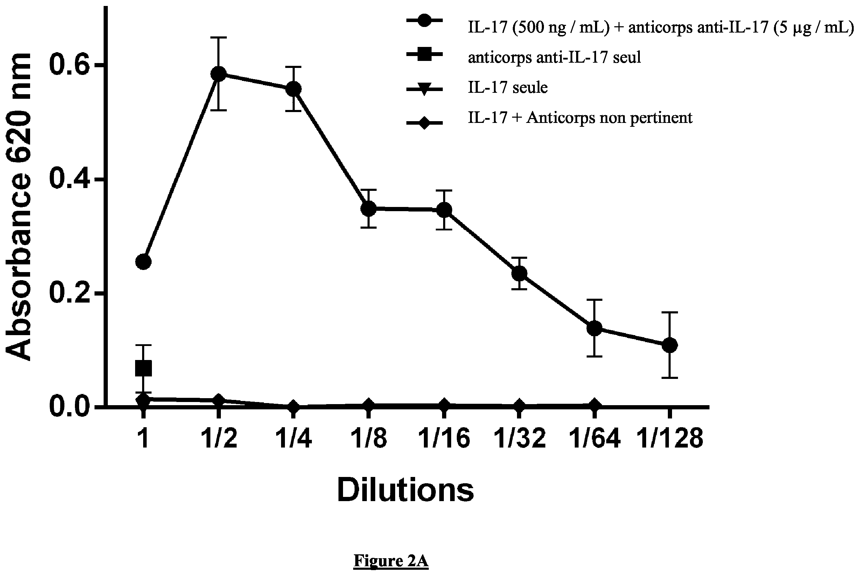

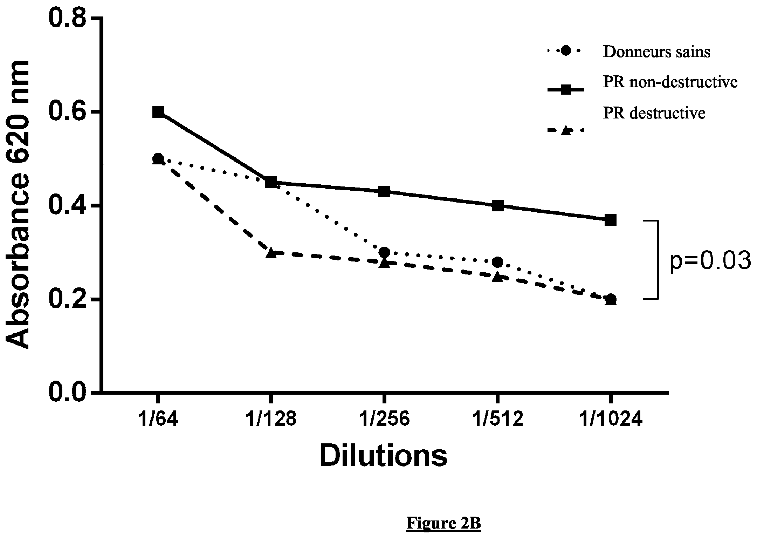

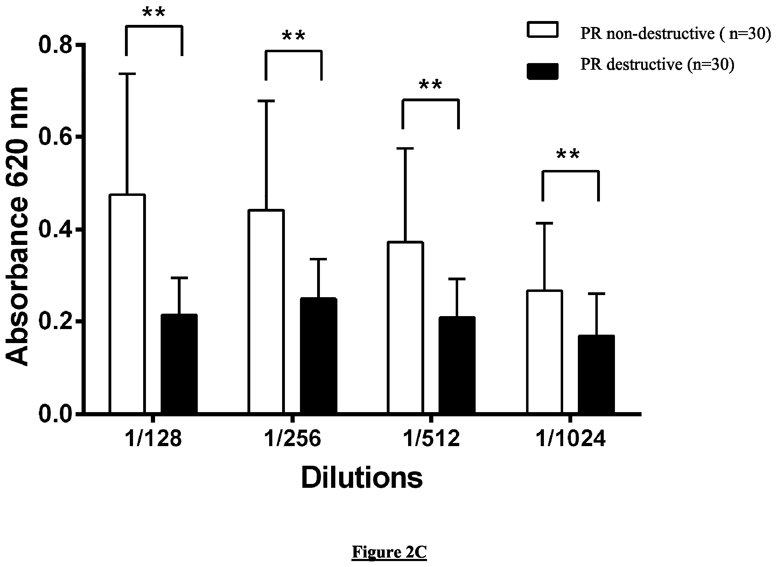

**Figure 2** **: Détection de complexes IL-17/anticorps anti-IL-17 dans le plasma de patients souffrant de polyarthrite rhumatoïde destructrice et non-destructrice, par rapport à des donneurs sains.**

Une courbe standard à huit points est déterminée à partir de dilutions en série (2-fold) et testée avec le contrôle positif formé *in vitro* à partir de 500 ng de IL-17 et 5 µg/ml d'anticorps humain anti-IL1-17 humaine **(A).**

Les complexes immuns (CI) sont détectés dans le plasma de 30 donneurs sains et de 60 patients atteints de polyarthrite rhumatoïde (PR) avec ou sans destruction osseuse. Pour chaque échantillon de plasma, une courbe standard de dilutions en série (2-fold) a été testée poiur déterminer les titres de complexes immuns **(B).**

Le niveau moyen de complexes immuns [IL-17/auto-anticorps anti-IL-17] dans les groupes PR non destructeur vs. PR destructeur sont représentés **(C).** Le test de Mann-Whitney montre une différence (p value) statistiquement significative (* < 0.05 ; *** < 0.001). Les barres d'erreur représentent la déviation standard.

### DESCRIPTION DETAILLEE DE L'INVENTION

De manière surprenante et inattendue, les inventeurs ont identifié la présence d'auto-anticorps anti-IL-17 chez des individus atteints de polyarthrite rhumatoïde.

De manière également surprenante, les inventeurs ont montré que la production d'auto-anticorps anti-IL-17 et/ou de complexes immuns [IL-17/auto-anticorps anti-IL-17] était plus courante chez des individus atteints de polyarthrite rhumatoïde non-destructrice que ceux atteints de polyarthrite rhumatoïde destructrice.

Cette relation négative entre la présence d'auto-anticorps anti-IL-17 et le phénomène de destruction osseuse dans cette maladie auto-immune et inflammatoire chronique suggère que ces auto-anticorps et complexes [IL-17/auto-anticorps anti-IL-17] protègent de la destruction osseuse, en neutralisant une tout ou partie de la fonction de l'Interleukine 17 (IL-17).

L'IL-17 ou IL17 désigne l'interleukine-17 telle qu'identifiée en 1993 par l'équipe de Rouvier *et al.* Elle a été rebaptisée en IL-17A après l'identification de nouveaux membres de la famille identifiés, dénommées IL-17B à IL-17F. L'IL-17 est une glycoprotéine homodimérique de 155 acides aminés, d'un poids de 35 kDa. L'IL-17 est sécrétée par des lymphocytes CD4+ et CD8+ et est impliquée dans la coordination de l'inflammation locale des tissus, notamment via l'induction de sécrétion de cytokines pro-inflammatoires et induisant la mobilisation des neutrophiles (Kolls and Linden, Immunity 2004, 21(4):467-476).

En effet, l'IL-17 induit la sécrétion de facteurs pro-inflammatoires par ses nombreuses cellules cibles, chaque type de cellules cibles étant spécialisé dans la production et sécrétion d'une ou plusieurs cytokines ou chemokines.

La présente invention concerne tout particulièrement l'IL-17A, mais peut également être appliquée à d'autres membres de la famille : IL-17B, IL-17C, IL-17D, IL-17E et en particulier IL-17F qui présente le plus fort taux d'identité de séquence avec l'IL-17A. Par analogie, les anticorps, auto-anticorps anti-IL-17A, et complexes comprenant les dits auto-anticorps sont tout particulièrement considérés, bien que ceux spécifiquement dirigés contre les autres membres soient également pertinents d'un point de vue clinique (diagnostic, pronostic, et/ou thérapeutique).

Certes, il était connu que des maladies auto-immunes chroniques pouvaient être caractérisées par la présence d'auto-anticorps. Il était également connu que l'Interleukine 17 (IL-17) pouvait constituer une cible thérapeutique à l'égard d'un nombre croissant de maladies inflammatoires.

Ainsi, des essais cliniques pour le traitement de la polyarthrite rhumatoïde mettant en oeuvre des anticorps anti-IL17 ont déjà été rapportés, notamment par Hueber et al. (Sci Transl Med. 2010; 2: 52ra72) et Genovese et al. (Arthritis Rheumatol. 2014; 66: 1693-1704). Toutefois, les résultats issus de ces essais cliniques sont hétérogènes.

Sans vouloir être lié à la théorie, les inventeurs sont d'avis que cette hétérogénéité est liée à la présence, à des niveaux variables, d'autoanticorps anti-IL17 et/ou de complexes immuns [IL-17/auto-anticorps anti-IL-17].

En particulier, les résultats obtenus (voir exemples) sur des échantillons biologiques issus de patients suggèrent que, dans la polyarthrite rhumatoïde non destructive, ces auto-anticorps anti-IL17 sont présents en excès et lient l'interleukine IL-17 pour former davantage de complexes [IL-17/auto-anticorps anti-IL-17] ; la conséquence, observée, est ainsi une diminution voire une absence de niveaux détectables de l'IL-17 bioactive libre.

La détection d'autoanticorps anti-IL17 et/ou de complexes immuns [IL-17/auto-anticorps anti-IL-17] chez ces individus représente donc un biomarqueur d'intérêt pour prédire le pronostic de ces maladies auto-immunes et inflammatoires chroniques, notamment pour prédire une destruction osseuse associée à ces maladies, mais aussi pour prédire une réponse à un traitement dans ce contexte.

On entend désigner par « *destruction osseuse* » une disparition progressive du tissu osseux due à l'état inflammatoire chronique du patient.

On entend désigner par « *polyarthrite rhumatoïde destructrice* », l'occurrence d'une polyarthrite rhumatoïde chez un individu, pour lequel une destruction osseuse est associée. Inversement, on entend désigner par « *polyarthrite rhumatoïde non destructrice* »*,* l'occurence d'une polyarthrite rhumatoïde chez un individu, pour lequel une destruction osseuse n'est pas associée.

Au regard des données expérimentales, une « *polyarthrite rhumatoïde non destructrice* » est statistiquement susceptible de correspondre à une polyarthrite rhumatoïde associée à la production d'auto-anticorps anti-IL17.

A l'inverse, une « *polyarthrite rhumatoïde destructrice* » est statistiquement susceptible de correspondre à une polyarthrite rhumatoïde non associée à la production d'auto-anticorps anti-IL17, ou pour laquelle la production d'auto-anticorps anti-IL17 est insuffisante.

La survenue d'une polyarthrite rhumatoïde destructrice ou non-destructrice peut être également déterminée à partir du score de Larsen, et tout particulièrement du score de Larsen au poignet (ou « wrist Larsen score »). Un score de Larsen au-dessus de 2 est généralement considéré comme caractéristique d'une polyarthrite rhumatoïde destructrice. Un score de Larsen compris entre 0 et 1 correspond généralement à une polyarthrite rhumatoïde non-destructrice. La signification et la détermination d'un score de Larsen, en particulier d'un score de Larsen au poignet, fait partie des connaissances générales de l'Homme du métier.

On entend en particulier désigner par « *détermination de la production d'auto-anticorps anti-IL17 »,* (i) le niveau d'un auto-anticorps anti-IL-17 et/ou (ii) le niveau d'un complexe [IL-17/auto-anticorps anti-IL-17].

Le terme « *anticorps* » est ici utilisé dans son sens le plus général, et couvre spécifiquement les anticorps monoclonaux (incluant les anticorps monoclonaux complets), les anticorps polyclonaux (ex. anticorps bi-spécifiques), et les fragments d'anticorps pour autant qu'ils présentent l'activité biologique désirée.

Le terme « *fragment d'anticorps* » désigne des fragments d'anticorps comprenant une partie d'un anticorps complet, généralement la partie responsable de la fixation à l'antigène ou son domaine variable. Des exemples de fragments d'anticorps incluent les fragments Fab, Fab', F(ab')₂, et Fv ; les diabodies; anticorps linéaires; anticorps à chaîne unique ; et anticorps multi-spécifiques formés de fragments d'anticorps.

Le terme « *auto-anticorps* » désigne un anticorps produit par le système immunitaire de l'hôte lui-même et dirigé contre une ou plusieurs de ses protéines.

Le terme « *individu* » englobe tout individu, humain ou non-humain (de préférence humain) susceptible de développer une maladie auto-immune ou inflammatoire chronique.

Selon un premier mode de réalisation, l'invention concerne un procédé *in vitro* pour évaluer le pronostic d'une polyarthrite rhumatoïde chez un individu, comprenant les étapes suivantes :
a) déterminer (i) le niveau d'un auto-anticorps anti-IL-17 et/ou (ii) le niveau d'un complexe [IL-17/auto-anticorps anti-IL-17] dans un échantillon biologique dudit individu, et
b) comparer le niveau d'auto-anticorps et/ou de complexe déterminé à l'étape a) avec une valeur de référence, la dite comparaison étant indicative du pronostic de la polyarthrite rhumatoïde chez le dit individu. rhumatoïde peut être :
   - une polyarthrite rhumatoïde destructrice ;
   - une polyarthrite rhumatoïde non destructrice ;
   - une polyarthrite rhumatoïde chez un individu produisant des auto-anticorps anti-IL-17 ; ou
   - une polyarthrite rhumatoïde chez un individu ne produisant pas d'auto-anticorps anti-IL-17.

Plus spécifiquement, le procédé *in vitro* tel que défini ci-dessus peut être mis en oeuvre pour
(i) déterminer le risque de destruction osseuse chez un individu affecté de la polyarthrite rhumatoïde; et/ou
(ii) déterminer les chances de réponse d'un individu atteint de la polyarthrite rhumatoïde à un traitement comprenant l'administration d'un principe actif inhibiteur de l'IL-17 (notamment un anticorps anti-IL-17); et/ou
(iii) déterminer l'efficacité d'un traitement ou d'une prévention d'une destruction osseuse d'un individu atteint de la polyarthrite rhumatoïde, ledit traitement ou la dite prévention consistant en l'administration d'un principe actif inhibiteur de l'IL-17 (notamment un anticorps anti-IL-17).

Ainsi, selon ces modes particuliers de réalisation la comparaison à l'étape b) est indicative du dit risque de destruction osseuse, des dites chances de réponse au dit traitement, et/ou de l'efficacité du dit traitement ou de la dite prévention d'une destruction osseuse.

La présente divulgation décrit un procédé *in vitro* de sélection d'un individu chez qui la présence d'auto-anticorps anti-IL-17 traduit la présence dans un échantillon biologique (dont le sang) du dit individu de cellules productrices de cet anticorps. Cette présence de cellules productrices d'auto-anticorps anti-IL-17 autorise leur isolement à partir du dit échantillon, et notamment à partir d'un prélèvement de sang.

L'utilisation de techniques actuelles de laboratoire permet ensuite d'isoler le dit auto-anticorps anti-IL-17, notamment pour un usage thérapeutique.

En particulier, ce procédé *in vitro* d'évaluation peut être mis en œuvre dans un procédé *in vitro* de sélection d'anticorps pour traiter (i) une polyarthrite rhumatoïde et/ou (ii) une destruction osseuse associée à la dite maladie, comprenant les étapes suivantes :
a) déterminer le niveau (i) d'un auto-anticorps anti-IL-17 et/ou (ii) d'un complexe [IL-17/auto-anticorps anti-IL-17] dans un échantillon biologique d'un individu évalué selon les procédés décrits ci-dessus, et/ou la revendication 1 ;
b) sélectionner un auto-anticorps anti-IL-17 ou une cellule productrice d'un auto-anticorps anti-IL-17 à partir d'un échantillon biologique du dit individu ; le dit auto-anticorps étant apte à traiter la dite maladie, et/ou la dite destruction osseuse.

Les échantillons biologiques des étapes **a)** et **b)** du dit procédé de sélection peuvent être identiques ou différents. L'étape **b)** de sélection peut être réalisée à partir de tout échantillon biologique susceptible de comprendre le dit auto-anticorps anti-IL-17 ou la dite cellule productrice, ce qui inclut : le sérum, le plasma, le sang et les organes lymphoïdes primaires ou secondaires.

Ainsi, l'invention concerne un procédé *in vitro* de sélection d'anticorps pour traiter (i) une polyarthrite rhumatoïde, et/ou (ii) une destruction osseuse associée à la dite maladie, comprenant les étapes suivantes :
**a)** déterminer le niveau (i) d'un auto-anticorps anti-IL-17 et/ou (ii) d'un complexe [IL-17/auto-anticorps anti-IL-17] dans un échantillon biologique d'un individu atteint d'une polyarthrite rhumatoïde
**b)** comparer le niveau d'auto-anticorps et/ou de complexe déterminé à l'étape a) avec une valeur de référence;
**c)** sélectionner un auto-anticorps anti-IL-17 ou une cellule productrice d'un auto-anticorps anti-IL-17 à partir d'un échantillon biologique du dit individu; le dit auto-anticorps étant apte à traiter la dite maladie, et/ou la dite destruction osseuse.

Dans le cadre des dits procédés *in vitro* de sélection d'anticorps, l'échantillon biologique peut être en particulier issu d'un individu de préférence atteint de polyarthrite rhumatoïde non destructrice.

Selon un second mode de réalisation, l'invention concerne un procédé *in vitro* pour distinguer une polyarthrite rhumatoïde (PR) destructrice d'une polyarthrite rhumatoïde non destructrice chez un individu, comprenant les étapes suivantes :
**a)** déterminer (i) le niveau d'un auto-anticorps anti-IL-17 et/ou (ii) le niveau d'un complexe [IL-17/auto-anticorps anti-IL-17] dans un échantillon biologique dudit individu, et
**b)** comparer le niveau d'auto-anticorps et/ou de complexe déterminé à l'étape a) avec une valeur de référence, la dite comparaison étant indicative d'une polyarthrite rhumatoïde (PR) destructrice ou non destructrice chez le dit individu.

Selon un troisième mode de réalisation, l'invention concerne un anticorps anti-IL-17 isolé, pour le traitement ou la prévention (i) d'une polyarthrite rhumatoïde destructrice et/ou (ii) d'une polyarthrite rhumatoïde non destructrice et/ou (iii) d'une polyarthrite rhumatoïde chez un individu produisant des auto-anticorps anti-IL-17 et/ou (iv) d'une polyarthrite rhumatoïde chez un individu ne produisant pas des auto-anticorps anti-IL-17, et/ou (v) d'une destruction osseuse à l'égard de l'un quelconque des groupes (i) à (iv).

Selon un quatrième mode de réalisation, l'invention concerne un anticorps anti-IL-17 humain isolé, obtenu à partir d'un échantillon biologique d'un individu atteint de polyarthrite rhumatoïde et produisant des auto-anticorps anti-IL-17.

Un anticorps anti-IL-17 isolé selon l'invention est, de préférence, un anticorps isolé à partir du procédé *in vitro* de sélection de l'invention, et/ou un anticorps isolé à partir d'un échantillon biologique d'un individu atteint de polyarthrite rhumatoïde, et de préférence atteint de polyarthrite rhumatoïde non destructrice.

En particulier, le dit anticorps anti-IL17 peut être obtenu selon un procédé comprenant les étapes suivantes :
a) préparer une composition enrichie en lymphocytes producteurs d'anticorps anti-IL17 à partir d'un échantillon provenant d'un individu atteint de polyarthrite rhumatoïde, et de préférence atteint de polyarthrite rhumatoïde non destructrice,
b) sélectionner au moins un lymphocyte B producteur d'anticorps anti-IL17, ou au moins un clone de lymphocytes B producteurs d'anticorps anti-IL17, et
c) obtenir le dit anticorps anti IL17.

Dans certains modes de réalisation, l'échantillon à partir duquel est préparée la composition enrichie en lymphocytes producteurs d'anticorps anti-IL17, à l'étape a), est un échantillon provenant de sang humain, ce qui englobe le sang total et une fraction du sang enrichie en cellules telles qu'une fraction enrichie en cellules mononuclées du sang périphérique et une fraction enrichies en lymphocytes.

L'étape b) peut être réalisée selon toute technique bien connue de l'homme du métier.

L'étape c) peut être réalisée par culture du ou des lymphocytes(s) B sélectionnés à l'étape b), puis purification de l'anticorps anti-IL17 ainsi produit.

Le plus couramment, le ou les lymphocytes B sélectionnés à l'étape b) sont ensuite immortalisés sous forme d'une lignée cellulaire productrice du dit anticorps anti-IL17. Puis les cellules en lignée sont cultivées et l'anticorps anti-IL17 ainsi produit est ensuite purifié.

### Procédés in vitro

Aux fins de réaliser les procédés *in vitro* décrits ci-dessus, on entend par « *valeur de référence* », un niveau d'auto-anticorps anti-IL-17 et/ou (ii) niveau de complexe [IL-17/auto-anticorps anti-IL-17] déterminé chez des individus/patients affectés d' une polyarthrite rhumatoïde, et dont la survenue ou non d'une destruction osseuse est éventuellement connue, ou pour lequel un phénomène de destruction osseuse a été le cas échéant observé.

En général, ladite valeur de référence est une valeur moyenne mesurée à partir d'un échantillon biologique d'une pluralité d'individus affectés d'une polyarthrite rhumatoïde, et dont la survenue (ou non) d'une destruction osseuse, ou encore dont la production des dits niveaux d'auto-anticorps anti-IL-17 et/ou (ii) niveau de complexe [IL-17/auto-anticorps anti-IL-17] est connue.

Aux fins de mise en œuvre des procédés selon l'invention, on peut choisir comme « *valeur de référence* » une valeur moyenne déterminée ou mesurée chez des patients affectés d'une polyarthrite rhumatoïde, et dont la survenue ou non d'une destruction osseuse est observée.

Dans certains modes de réalisation du procédé, la valeur de référence est une valeur déterminée chez des individus ne présentant pas de destruction osseuse et/ou chez des individus dont la polyarthrite rhumatoïde est associée à la production d'auto-anticorps anti-IL-17 et/ou de complexe [IL-17/auto-anticorps anti-IL-17]. Dans ces modes de réalisation, un patient testé selon le procédé de l'invention sera classé « *à risque réduit ou nul de destruction osseuse* » lorsque le (i) niveau d'auto-anticorps anti-IL-17 et/ou (ii) niveau de complexe [IL-17/auto-anticorps anti-IL-17] est supérieur à la dite valeur de référence.

Dans certains modes de réalisation du procédé, la valeur de référence est une valeur déterminée chez des patients présentant une destruction osseuse et/ou chez des individus dont la polyarthrite rhumatoïde n'est pas associée à la production d'auto-anticorps anti-IL-17 et/ou de complexe [IL-17/auto-anticorps anti-IL-17].

Dans ces modes de réalisation, un patient testé selon le procédé de l'invention sera classé « *à risque modéré ou important de destruction osseuse* » lorsque le (i) niveau d'auto-anticorps anti-IL-17 et/ou (ii) niveau de complexe [IL-17/auto-anticorps anti-IL-17] est inférieur à la dite valeur de référence.

Dans certains modes de réalisation du procédé, la valeur de référence est une valeur déterminée chez un même patient, avant ou après administration d'un principe actif au dit patient, ou encore avant ou après mise en contact de l'échantillon biologique avec le dit principe actif. Ce mode de réalisation peut être avantageusement mis en valeur pour déterminer :
- les chances de réponse d'un individu atteint de la polyarthrite rhumatoïde à un traitement comprenant l'administration d'un principe actif inhibiteur de l'IL-17; et/ou
- l'efficacité d'un traitement ou d'une prévention d'une destruction osseuse d'un individu atteint de la dite maladie, ledit traitement ou la dite prévention consistant en l'administration d'un principe actif inhibiteur de l'IL-17.

L'étape d'administration d'un principe actif, ou composé candidat, sera réalisée sur une période adéquate pour juger de l'efficacité dudit composé, cette période allant de un jour à plusieurs mois, et étant calquée sur les périodes d'administration habituelles de ce type de composés.

Ainsi, selon un mode particulier de réalisation, l'invention a trait à un procédé *in vitro* pour évaluer le pronostic d'une polyarthrite rhumatoïde chez un individu, pour déterminer :
- les chances de réponse du dit individu à un traitement comprenant l'administration d'un principe actif inhibiteur de l'IL-17; et/ou
- l'efficacité d'un traitement ou d'une prévention d'une destruction osseuse du dit individu, ledit traitement ou la dite prévention consistant en l'administration d'un principe actif inhibiteur de l'IL-17 ; et comprenant les étapes suivantes :
   **a)** déterminer (i) le niveau d'un auto-anticorps anti-IL-17 et/ou (ii) le niveau d'un complexe [IL-17/auto-anticorps anti-IL-17] dans un échantillon biologique dudit individu avant la dite administration ;
   **b)** administrer à l'individu le dit principe actif inhibiteur de l'IL-17 ;
   **c)** déterminer (i) le niveau d'un auto-anticorps anti-IL-17 et/ou (ii) le niveau d'un complexe [IL-17/auto-anticorps anti-IL-17] dans un échantillon biologique dudit individu après la dite administration ; et
   **d)** comparer le niveau d'auto-anticorps et/ou de complexe déterminé aux étapes a) et c) avec une valeur de référence, la dite comparaison étant indicative des dites chances de réponse et/ou de l'efficacité du dit traitement ou prévention.

Il est entendu que ledit principe actif inhibiteur de l'IL-17 (qui est de préférence un anticorps anti-IL-17) est jugé efficace lorsque la valeur déterminée à l'étape a) est supérieure à la valeur déterminée à l'étape c).

Dans encore d'autres modes de réalisation, la valeur de référence est une valeur dite « *seuil* » ou de « *cut-off* »*,* qui est déterminée à partir (i) de valeurs déterminées chez des patients « *à risque réduit ou nul* » et (ii) de valeurs déterminées chez des patients « *à risque modéré ou important*»*.* Dans ces modes de réalisation, un patient testé selon le procédé de l'invention sera classé « *à risque réduit ou nul de destruction osseuse* » lorsque le (i) niveau d'auto-anticorps anti-IL-17 et/ou (ii) niveau de complexe [IL-17/auto-anticorps anti-IL-17] mesuré pour ce patient pour ce patient est supérieure à la valeur de référence.

Une valeur de référence « *seuil* » ou de « *cut-off* » peut être aisément déterminée par l'homme du métier à l'aide de ses connaissances générales. Une valeur de référence « seuil » ou de « cut-off » peut être par exemple déterminée comme décrit par Limmathurotsakul et al. (2011, Clin. Infect. Dis., Vol. 52 : 1024-1028).

Il est entendu que pour la mise en œuvre de tous ces procédés, l'échantillon biologique a été prélevé sur l'individu testé, et qu'il s'agit d'un échantillon de sang total, de plasma, de sérum, de liquide synovial, de liquide céphalo-rachidien, de liquide pleural, ou de liquide péritonéal dudit individu.

Lesdits procédés *in vitro* pourront aussi être réalisés sur tout type de cellules susceptibles de produire les dits auto-anticorps, ce qui inclut les lymphocytes B issus d'individus ayant une maladie auto-immune ou inflammatoire chronique, par exemple une polyarthrite rhumatoïde.

Lorsque l'échantillon biologique comprend ou est constitué de cellules, ces procédés seront de préférence caractérisés en ce que les cellules utilisées sont des cultures primaires de cellules, notamment de cellules prélevées sur l'individu dont l'échantillon biologique est testé.

Selon un mode de réalisation particulier de ces procédés *in vitro,* l'étape a) consiste à déterminer le niveau d'un auto-anticorps anti-IL-17 dans le dit échantillon biologique.

Selon un autre mode de réalisation particulier de ces procédés *in vitro,* l'étape **a)** consiste à déterminer le niveau d'un complexe [IL-17/auto-anticorps anti-IL-17] dans le dit échantillon biologique.

En particulier, l'échantillon biologique peut être choisi parmi : du sang total, du plasma, et du sérum.

Selon un mode de réalisation préféré, l'auto-anticorps anti-IL-17 est un auto-anticorps anti-IL-17A ; et le complexe [IL-17/auto-anticorps anti-IL-17] est un complexe [IL-17A/auto-anticorps anti-IL-17A].

Ainsi, l'auto-anticorps anti-IL-17 peut être, de manière préférée, un auto-anticorps humain dirigé contre l'interleukine IL-17A humaine.

La détermination du niveau d'un auto-anticorps anti-IL-17 et/ou (ii) le niveau d'un complexe [IL-17/auto-anticorps anti-IL-17] dans un échantillon biologique peut être réalisée par tout test connu de l'homme du métier. De manière non exhaustive, des tests pour la détermination d'auto-anticorps sont référencés dans Aggarwal et al (Best Practice & Research Clinical Rheumatology 28 (2014) 907-920)..

Par exemple, la détermination du niveau d'un auto-anticorps anti-IL-17 et/ou (ii) le niveau d'un complexe [IL-17/auto-anticorps anti-IL-17] peut être réalisée selon l'une des méthodes suivantes : immuno-électrophorèse, contre-électrophorèse, immunodiffusion sur gel, immuno-agglutination, immunofluorescence, ELISA, néphélémétrie et immuno-blotting.

Selon un mode de réalisation, l'étape a) est mise en œuvre sous la forme d'un test ELISA. Les tests immunologiques de quantification sous la forme de tests ELISA sont connus de l'homme du métier. Ils peuvent être notamment mis en œuvre sous la forme d'ELISA compétitifs (voir exemples et figure 1A) ou d'ELISA indirects (voir figure 1B).

Selon un sous-mode de réalisation, un test ELISA compétitif peut être mis en œuvre pour déterminer le niveau d'un auto-anticorps anti-IL-17 dans un échantillon.

Selon un sous-mode de réalisation, un test ELISA indirect peut être mis en œuvre pour déterminer le niveau d'un complexe [IL-17/auto-anticorps anti-IL-17] dans un échantillon.

Ainsi, un test ELISA compétitif pour déterminer le niveau d'un auto-anticorps anti-IL-17 dans un échantillon peut comprendre les étapes suivantes :
**a)** optionnellement mettre en contact un échantillon susceptible de contenir un auto-anticorps anti-IL17, en présence d'un agent susceptible d'inhiber l'interaction de facteur rhumatoïde éventuellement présent avec les dits auto-anticorps (par exemple un sérum issu d'un autre organisme) ;
**b)** mettre en contact le dit échantillon avec une interleukine IL-17 pendant un temps suffisant pour que les dits auto-anticorps anti-IL-17 interagissent avec la dite interleukine ;
**c)** mettre en contact le dit échantillon avec un anticorps exogène dirigé contre la dite interleukine pendant un temps suffisant pour que le dit anticorps exogène entre en compétition avec les dits auto-anticorps anti-IL-17 pour interagir avec la dite interleukine ;
**d)** déterminer le niveau d'auto-anticorps anti-IL-17 dans le dit échantillon à partir du niveau d'anticorps exogène interagissant avec la dite interleukine.

En particulier, le dit anticorps exogène peut être sous forme marquée, afin de faciliter la détermination du niveau d'auto-anticorps en étape d).

L'étape d) peut en outre inclure une étape de fixation du dit échantillon biologique sur un support sur lequel sont fixés des anticorps anti-IL17.

Un test ELISA indirect pour déterminer le niveau d'un complexe [IL-17/auto-anticorps anti-IL-17] dans un échantillon peut comprendre les étapes suivantes :
**a)** mettre en contact un échantillon susceptible de contenir un complexe [IL-17/auto-anticorps anti-IL-17] avec un support sur lequel sont fixés des anticorps anti-IL17, pendant un temps suffisant pour que lesdits complexes interagissent avec le dit anticorps. ;
**b)** mettre en contact le dit échantillon avec un anticorps exogène dirigé contre le dit auto-anticorps anti-IL-17, pendant un temps suffisant pour que le dit anticorps exogène interagisse avec le dit auto-anticorps anti-IL-17.
**c)** déterminer le niveau de complexe [IL-17/auto-anticorps anti-IL-17] dans le dit échantillon à partir du niveau d'anticorps exogène interagissant avec le dit auto-anticorps.

En particulier, le dit anticorps exogène peut être sous forme marquée, afin de faciliter la détermination du niveau

Les dits échantillons peuvent tout particulièrement être des échantillons de sang, ou dérivés du sang, tels que du plasma ou du sérum.

Selon un mode de réalisation, un procédé *in vitro* selon l'invention peut comprendre, en outre, une étape de détermination, dans un échantillon biologique du dit individu, du niveau d'un polypeptide choisi parmi : les cytokines l'IL-17A, IL-17F, IL-25, IL-23, les facteurs de transcription, dont RORγt, et les récepteurs à l'interleukine IL-17 tels que IL-17RA, IL-17RB, IL-17RC, les auto-anticorps.

Ainsi, selon un mode de réalisation particulier, un procédé *in vitro* selon l'invention peut comprendre une étape de détermination, dans un échantillon biologique du dit individu, du niveau d'un polypeptide choisi parmi : les cytokines IL-17A, IL-17F, IL-25, IL-23, le facteur de transcription RORγt, et les récepteurs IL-17RA, IL-17RB, IL-17RC, les auto-anticorps dirigés contre IL-1α, les auto-anticorps dirigés contre IL-8 et/ou les auto-anticorps dirigés contre l'ostéopontine.

### Composition pharmaceutique et applications thérapeutiques

Des anticorps anti-IL-17 en tant que tels, et pour le traitement ou la prévention de maladies auto-immunes ou inflammatoires chroniques sont décrits ci-dessous.

Ces anticorps anti-IL-17 peuvent être mis en œuvre en tant que médicaments, ou encore utilisés pour la préparation d'un médicament.

La mise en évidence d'auto-anticorps anti-IL-17 et de complexes [IL-17/auto-anticorps anti-IL-17] chez des patients atteints de polyarthrite rhumatoïde destructrice et non destructrice permet également d'envisager l'administration d'anticorps anti-IL-17 à des sous-groupes particuliers d'individus atteints de :
- polyarthrite rhumatoïde destructrice ;
- polyarthrite rhumatoïde non destructrice ;
- polyarthrite rhumatoïde associée à la production d'auto-anticorps anti-IL-17 ;
- polyarthrite rhumatoïde non associée à la production d'auto-anticorps anti-IL-17 anti-IL-17 ; et/ou
- d'une destruction osseuse associée à l'un quelconque des sous-groupes précédents.

Ainsi, la présente invention concerne un anticorps anti-IL-17 isolé pour le traitement ou la prévention (i) d'une polyarthrite rhumatoïde destructrice et/ou (ii) d'une polyarthrite rhumatoïde non destructrice et/ou (iii) d'une polyarthrite rhumatoïde chez un individu produisant des auto-anticorps anti-IL-17 et/ou (iv) d'une polyarthrite rhumatoïde chez un individu ne produisant pas des auto-anticorps anti-IL-17, et/ou (v) d'une destruction osseuse associée à l'un quelconque des groupes (i) à (iv).

La présente invention concerne également l'utilisation d'un anticorps anti-IL-17 isolé pour la préparation d'un médicament pour le traitement ou la prévention (i) d'une polyarthrite rhumatoïde destructrice et/ou (ii) d'une polyarthrite rhumatoïde non destructrice et/ou (iii) d'une polyarthrite rhumatoïde chez un individu produisant des auto-anticorps anti-IL-17 et/ou (iv) d'une polyarthrite rhumatoïde chez un individu ne produisant pas d'auto-anticorps anti-IL-17, et/ou (v) d'une destruction osseuse associée à l'un quelconque des groupes (i) à (iv).

De préférence, les sous-groupes d'individus à considérer dans le cadre d'une administration d'anticorps anti-IL-17 et/ou de la préparation d'un médicament, sont ceux atteints de :
- polyarthrite rhumatoïde destructrice ;
- polyarthrite rhumatoïde non associée à la production d'auto-anticorps anti-IL-17; et/ou
- d'une destruction osseuse associée à l'un quelconque des sous-groupes précédents.

Les dits sous-groupes d'individus peuvent notamment être ceux évalués par l'un quelconque des procédés *in vitro* d'évaluation selon l'invention.

Les dits anticorps anti-IL-17 selon l'invention peuvent notamment être ceux sélectionnés par l'un quelconque des procédés *in vitro* de sélection selon l'invention.

Un anticorps anti-IL-17 selon l'invention peut être un anticorps anti-IL1-7 obtenu à partir d'un échantillon biologique d'un individu produisant des auto-anticorps anti-IL-17.

En particulier, le dit anticorps anti-IL-17 est un anticorps anti-IL-17 humain isolé, obtenu à partir d'un échantillon biologique d'un individu produisant des auto-anticorps anti-IL-17.

Un anticorps anti-IL-17 peut aussi être issu d'une cellule productrice d'auto-anticorps anti-IL-17; la dite cellule productrice (par exemple un lymphocyte B et/ou un hybridome) étant obtenue à partir d'un échantillon biologique d'un individu produisant les dits auto-anticorps anti-IL-17.

Le dit individu duquel est isolé le dit auto-anticorps anti-IL-17 ou la dite cellule productrice, est atteint de polyarthrite rhumatoïde.

Selon un mode de réalisation, l'anticorps anti-IL-17 est un anticorps anti-IL-17 humain isolé, obtenu à partir d'un échantillon biologique d'un individu atteint de polyarthrite rhumatoïde et produisant des auto-anticorps anti-IL-17.

Selon un mode de réalisation exemplifié, l'anticorps anti-IL-17 est un anticorps humain dirigé contre l'interleukine IL-17 humaine obtenu après immortalisation de lymphocytes B humains issus de cellules PBMC (« *Peripheral blood mononuclear cells* ») d'individus atteints de polyarthrite rhumatoïde et produisant des auto-anticorps anti-IL-17.

De préférence, les dits anticorps anti-IL-17 sont des anticorps monoclonaux.

L'anticorps anti-IL-17 peut être un anticorps humain dirigé contre l'interleukine (IL17A) humaine, l'IL-17F humaine, ou dirigé à la fois contre l'IL-17A humaine et l'IL-17F humaine.

Selon un mode de réalisation préféré, l'anticorps anti-IL-17 est un anticorps anti-IL-17A.

Un tel anticorps peut être identique ou substantiellement identique à un anticorps naturel, par exemple produit à partir de cellules productrices d'anticorps, telles que des lymphocytes B issus d'individus atteints de polyarthrite rhumatoïde, tels que des individus atteints de polyarthrite rhumatoïde non destructrice et/ou de polyarthrite rhumatoïde associée à la production d'auto-anticorps anti-IL-17.

Ainsi, l'auto-anticorps anti-IL-17 peut être, de manière préférée, un auto-anticorps humain, dirigé contre l'interleukine IL-17A humaine l'IL-17F humaine, ou dirigé à la fois contre l'IL-17A humaine et l'IL-17F humaine.

Un anticorps anti-IL-17 isolé tel que défini ci-dessus peut être mis en œuvre dans une composition pharmaceutique et/ou en tant que médicament.

L'invention concerne donc également une composition pharmaceutique ou un médicament comprenant le dit anticorps anti-IL-17.

L'invention est définie par les revendications annexées.

### EXEMPLES

### MATÉRIEL ET MÉTHODES

***Patients :*** 60 patients atteints de polyarthrite rhumatoïde (PR) ont été sélectionnés dans une large base de données, et classifiés en deux groupes selon un ratio 1 :1, à partir de leur degré radiographique de destruction (Larsen score). La polyarthrite rhumatoïde (PR) destructrice est définie par un score Larsen du poignet supérieur à 2, et une PR non destructrice entre 0 et 1. Tous les paramètres relatifs à la PR ont été obtenus depuis la base de données clinique. Les patients PR destructeur et non destructeur ont été appariés en fonction du sexe (femme/homme = 21/09 vs. 20/10), âge (66.1 ± 10.8 vs. 71.3 ± 9.3 years), durée de la maladie (18.6 ± 9.6 vs. 23.0 ± 9.8 années), DAS28 (3.9 ± 1.2 vs. 4.0 ± 1.4), à l'exception du score Larsen (0.5 ± 0.5 vs. 3.3 ± 1.0, p <0.0001) **(Tableau 1).** Trente donneurs sains ont été utilisés comme contrôles négatifs. Un consentement écrit a été obtenu de chaque sujet. Le protocole est en accord avec le comité d'éthique des hôpitaux de Lyon.

***Détection d'anticorps anti-IL-17 par un test ELISA compétitif (******Figure 1A******):*** le plasma de patients PR et de donneurs sains est d'abord pré-incubé avec du sérum de cheval sur la nuit pour prévenir les réactions croisées avec le facteur rhumatoïde (RF). Des échantillons de plasma aux dilutions 1/4, 1/8 et 1/16 sont ensuite incubés avec 30 µl de IL-17A (50 ng/ml) (IL17A, Dendritics, Lyon, France). Après 1h d'incubation, un anticorps de détection anti IL-17 humaine (406G9.02-HRP, Dendritics, Lyon, France) a été rajouté. Ce mélange est transféré dans une plaque de 96 puits comprenant un anticorps de souris anti-IL-17 humaine (408H6.01, Dendritics, Lyon, France) pendant 2h. Le substrat Tetramethylbenzidine (TMB) a été ajouté et l'absorbance à 620 nm est déterminée.

***Détection de complexes IL-17*/*anticorps anti-IL-17 par un test ELISA indirect (******Figure 1B******)*** : les puits sont incubés sur la nuit avec 3 µg/mL d'un anticorps de capture anti-IL-17 (408H6.01). Après lavage au PBS/Tween 0.05 %, 100 µl de plasma dilués au ¼ sont ajoutés sur la nuit (première dilution au ½ dans du sérum de cheval et seconde dilution dans un mélange PBS/BSA/Tween). Après 3 lavages, un anticorps de chèvre anti-Fragment Fc d'IgG humaine (anti-IgG Fc humaine) conjugué à la peroxydase (109-035-098, Jackson Immuno research, Baltimore, USA) au 1/5000 ème est ajouté et incubé pendant 1h30. Comme contrôle positif, un mélange d'un anticorps humain anti-IL-17A humaine est isolé du sang d'un patient atteint de polyarthrite rhumatoïde (5µg/ml) et une interleukine IL-17 à 500 ng/ml a été utilisée. Cet anticorps humain dirigé contre l'interleukine IL-17 humaine a été obtenu après immortalisation de lymphocytes B humains issus de cellules PBMC de patients atteints de polyarthrite rhumatoïde et après immortalisation par EBV et le système CD40 (DDXK-HuBBB, Dendritics, Lyon, France). Afin de vérifier l'élimination du facteur rhumatoïde, la réaction a été testée avec et sans sérum de cheval.

Analyse statistique: les données ont été exprimées en tant que moyenne ± SD. Un test (t-test) bilatéral non-paramétrique du logiciel Graphpad Prism a été utilisé. Une valeur *p* (*p* value) inférieure à 0.05 est considérée comme statistiquement significative.

### EXEMPLE 1 - Forte incidence d'auto-anticorps anti-IL-17 chez des patients atteints de polyarthrite rhumatoïde non destructive.

Un test compétitif d'ELISA a été développé pour mesurer les auto-anticorps anti-IL17 dans le plasma. Afin de prévenir la cross-réactivité avec le facteur rhumatoïde présent en larges quantités dans le plasma, une étape de pré-incubation en présence de sérum de cheval a été rajoutée. Un test contrôle positif utilisant des anticorps anti-IL-17 purifiés a montré une décroissance de l'absorbance avec la dilution (fig 1A), tandis que des anticorps non spécifiques ne montraient aucune variation.

Le plasma de 30 donneurs sains a permis d'identifier une valeur seuil. Il a été observé une absorbance à 0.9 ± 0.1 pour une dilution ½, et pas de variation pour des dilutions de ¼ à 1/8, ce qui indique une absence de positivité aux autoanticorps anti-IL-17.

A l'inverse, des anticorps anti-IL-17 ont été détectés pour 36.6 % des 60 patients atteints de polyarthrite rhumatoïde (*p* < 0.05 vs. controls), ce qui indique un lien entre la présence d'autoanticorps dirigés contre l'interleukine IL-17 et la polyarthrite rhumatoïde.

Afin d'étudier la relation entre la sévérité de la polyarthrite rhumatoïde, des plasmas issus d'individus atteints de polyarthrite rhumatoïde destructive et non-destructive. Les différents groupes sont appariés, comme indiqué dans la partie Matériel & Méthodes (voir **tableau 1 ci-dessous).** Les patients dits "non destructeur" présentaient cette pathologie depuis plusieurs années, afin de confirmer la sévérité réduite.

**Tableau 1: Paramètres cliniques des donneurs sains, polyarthrite rhumatoïde destructive et non-destructive, et incidence des autoanticorps anti-IL-17**

| Paramètres | Donneurs sains (n=30) | Patients PR (n=60) | Non-destructeur (n=30) | Destructeur (n=30) | *p* value |
|---|---|---|---|---|---|
| Sexe (F:M) | 20:10 | 41 :19 | 21:09 | 20:10 | n.s |
| Age (années) | 60.0 ± 5.5 | 68.7 ± 10.5 | 66.1 ± 10.8 | 71.3 ± 9.3 | n.s |
| Durée de la maladie (années) | | 20.8 ± 9.7 | 18.6 ± 9.6 | 23.0 ± 9.8 | n.s |
| DAS28 | | 4.0 ± 1.3 | 3.9 ± 1.2 | 4.0 ± 1.4 | n.s |
| Score Larsen | | 1.9 ± 0.8 | 0.5 ± 0.5 | 3.3 ± 1.0 | < 0.0001 |
| Facteur Rhumatoide positif (%) | | 63.2 | 57.1 | 69.2 | n.s |
| Anticorps Anti-CCP positif (%) | | 58.3 | 50.0 | 66.6 | n.s |
| Anticorps Anti-IL-17 (%) | 0.0 | 35.4 | **46.6** | **24.2** | < 0.05 |

| | | | | | |
|---|---|---|---|---|---|
| n.s = non significatif, F=femme, M=homme, Abs=anticorps, DAS28=score d'activité de la maladie ou Disease Activity Score 28. | | | | | |

Les anticorps anti-IL-17 ont été détectés dans 46,6 % des patients "non-destructeurs" alors que ces anticorps n'étaient détectés que dans 24,2 % des patients "destructeurs". Ces résultats suggèrent que les autoanticorps naturels anti-IL-17 sont associés à une polyarthrite rhumatoïde de meilleur pronostic, concernant la destruction osseuse.

Des observations similaires ont été rapportées pour d'autres cytokines. En particulier des niveaux augmentés d'auto-anticorps dirigés contre l'IL-1α chez des patients atteints de polyarthrite rhumatoïde suggèrent un rôle protecteur de ces anti IL-1α à l'égard de la destruction osseuse.

A l'inverse, des autoanticorps dirigés contre l'IL-8 et l'ostéopontine ont été associés à des manifestations extra-articulaires de la polyarthrite rhumatoïde.

## Revendications

1. Procédé *in vitro* pour évaluer le pronostic d'une maladie auto-immune ou inflammatoire chronique chez un individu, comprenant les étapes suivantes :
a) déterminer (i) le niveau d'un auto-anticorps anti-IL-17 et/ou (ii) le niveau d'un complexe [IL-17/auto-anticorps anti-IL-17] dans un échantillon biologique dudit individu, et
b) comparer le niveau d'auto-anticorps et/ou de complexe déterminé à l'étape a) avec une valeur de référence, la dite comparaison étant indicative du pronostic d'une maladie auto-immune ou inflammatoire chronique chez le dit individu ;
dans lequel la maladie auto-immune ou inflammatoire chronique est une polyarthrite rhumatoïde (PR).

2. Procédé *in vitro* selon la revendication 1, pour évaluer le pronostic d'une maladie auto-immune ou inflammatoire chronique chez un individu pour
(i) déterminer le risque de destruction osseuse chez le dit individu affecté de la dite maladie auto-immune ou inflammatoire chronique; et/ou
(ii) déterminer les chances de réponse du dit individu atteint de la dite maladie auto-immune ou inflammatoire chronique, à un traitement comprenant l'administration d'un principe actif inhibiteur de l'IL-17; et/ou
(iii) déterminer l'efficacité d'un traitement ou d'une prévention d'une destruction osseuse du dit individu atteint de la dite maladie auto-immune ou inflammatoire chronique, ledit traitement ou la dite prévention consistant en l'administration d'un principe actif inhibiteur de l'IL-17 ;
dans lequel la comparaison à l'étape **b)** est indicative du dit risque de destruction osseuse, des dites chances de réponse au dit traitement, et/ou de l'efficacité du dit traitement ou de la dite prévention d'une destruction osseuse.

3. Procédé *in vitro* de sélection d'anticorps pour traiter (i) une maladie auto-immune ou inflammatoire chronique, et/ou (ii) une destruction osseuse associée à la dite maladie, dans lequel la maladie auto-immune ou inflammatoire chronique est une polyarthrite rhumatoïde (PR), comprenant les étapes suivantes :
a) déterminer le niveau (i) d'un auto-anticorps anti-IL-17 et/ou (ii) d'un complexe [IL-17/auto-anticorps anti-IL-17] dans un échantillon biologique d'un individu évalué par le procédé selon la revendication 1 ;
b) sélectionner un auto-anticorps anti-IL-17 ou une cellule productrice d'un auto-anticorps anti-IL-17 à partir d'un échantillon biologique du dit individu ; le dit auto-anticorps étant apte à traiter la dite maladie, et/ou la dite destruction osseuse.

4. Procédé *in vitro* pour distinguer une polyarthrite rhumatoïde (PR) destructrice d'une PR non destructrice chez un individu, comprenant les étapes suivantes :
**a)** déterminer (i) le niveau d'un auto-anticorps anti-IL-17 et/ou (ii) le niveau d'un complexe [IL-17/auto-anticorps anti-IL-17] dans un échantillon biologique dudit individu, l'échantillon biologique étant choisi parmi du sang total, du plasma, et du sérum et
**b)** comparer le niveau d'auto-anticorps et/ou de complexe déterminé à l'étape a) avec une valeur de référence, ladite valeur de référence étant déterminée (i) chez des individus ne présentant pas de destruction osseuse et/ou dont la polyarthrite rhumatoïde est associée à la production d'auto-anticorps anti-IL-17 et/ou de complexe [IL-17/auto-anticorps anti-IL-17], ou (ii) chez des individus présentant une destruction osseuse et/ou chez des individus dont la polyarthrite rhumatoïde n'est pas associée à la production d'auto-anticorps anti-IL-17 et/ou de complexe [IL-17/auto-anticorps anti-IL-17], la dite comparaison étant indicative d'une polyarthrite rhumatoïde (PR) destructrice ou non destructrice chez le dit individu.

5. Procédé *in vitro* selon l'une des revendications précédentes, dans lequel l'étape **a)** consiste à déterminer le niveau d'un auto-anticorps anti-IL-17 dans le dit échantillon biologique.

6. Procédé *in vitro* selon l'une des revendications précédentes, dans lequel l'étape **a)** consiste à déterminer le niveau d'un complexe [IL-17/auto-anticorps anti-IL-17] dans le dit échantillon biologique.

7. Procédé *in vitro* selon l'une des revendications 1-3, dans lequel l'échantillon biologique est choisi parmi : du sang total, du plasma, du sérum, du liquide synovial, du liquide céphalo-rachidien, du liquide pleural, et du liquide péritonéal.

8. Procédé *in vitro* selon l'une des revendications précédentes, dans lequel l'auto-anticorps anti-IL-17 est un auto-anticorps anti-IL-17A ; et le complexe [IL-17/auto-anticorps anti-IL-17] est un complexe [IL-17A/auto-anticorps anti-IL-17A].

9. Procédé *in vitro* selon l'une des revendications précédentes, dans lequel l'étape a) est mise en œuvre sous la forme d'un test ELISA.

10. Procédé *in vitro* selon l'une des revendications précédentes, comprenant en outre, une étape de détermination, dans un échantillon biologique du dit individu, du niveau d'un polypeptide choisi parmi : les cytokines IL-17A, IL-17F, IL-25, IL-23, le facteur de transcription RORγt, et les récepteurs IL-17RA, IL-17RB, IL-17RC, les auto-anticorps dirigés contre IL-1α, les auto-anticorps dirigés contre IL-8 et/ou les auto-anticorps dirigés contre l'ostéopontine.

11. Un anticorps anti-IL-17 isolé, pour utilisation dans le traitement ou la prévention (i) d'une polyarthrite rhumatoïde destructrice ou (ii) d'une polyarthrite rhumatoïde chez un individu ne produisant pas d'auto-anticorps anti-IL-17, ou d'une destruction osseuse associée à l'un quelconque des groupes (i) ou (ii), ledit individu étant identifié par le procédé selon l'une des revendications 4 à 10.

## Patentansprüche

1. In-vitro-Verfahren zur Bewertung der Prognose einer chronischen Autoimmun- oder Entzündungskrankheit bei einem Individuum, umfassend die folgenden Schritte:
a) Bestimmen (i) des Niveaus eines Anti-IL-17-Auto-Antikörpers und/oder (ii) des Niveaus eines Komplexes [IL-17/Anti-IL-17-Auto-Antikörper] in einer biologischen Probe des Individuums, und
b) Vergleichen des in Schritt a) bestimmten Niveaus des Auto-Antikörpers und/oder des Komplexes mit einem Referenzwert, wobei der Vergleich die Prognose einer chronischen Autoimmun- oder Entzündungskrankheit bei dem Individuum anzeigt;
wobei es sich bei der chronischen Autoimmun- oder Entzündungskrankheit um rheumatoide Polyarthritis (PR) handelt.

2. In-vitro-Verfahren gemäß Anspruch 1 zur Bewertung der Prognose einer chronischen Autoimmun- oder Entzündungskrankheit bei einem Individuum zur
(i) Bestimmung des Risikos der Knochenzerstörung bei dem von der chronischen Autoimmun- oder Entzündungskrankheit betroffenen Individuum; und/oder
(ii) Bestimmung der Wahrscheinlichkeit einer Ansprechreaktion des Individuums mit der chronischen Autoimmun- oder Entzündungskrankheit auf eine die Verabreichung eines IL-17-hemmenden Wirkstoffs umfassende Behandlung; und/oder
(iii) Bestimmung der Wirksamkeit einer Behandlung oder einer Prävention einer Knochenzerstörung bei dem Individuum mit der chronischen Autoimmun- oder Entzündungskrankheit, wobei die Behandlung oder die Prävention aus der Verabreichung eines IL-17-hemmenden Wirkstoffs besteht;
wobei der Vergleich in Schritt b) das Risiko einer Knochenzerstörung, die Wahrscheinlichkeit einer Ansprechreaktion auf die Behandlung und/oder die Wirksamkeit der Behandlung oder der Prävention einer Knochenzerstörung anzeigt.

3. In-vitro-Verfahren zum Auswählen eines Antikörpers zum Behandeln (i) einer chronischen Autoimmun- oder Entzündungskrankheit und/oder (ii) einer mit der Krankheit assoziierten Knochenzerstörung, wobei es sich bei der chronischen Autoimmun- oder Entzündungskrankheit um eine rheumatoide Polyarthritis (PR) handelt, umfassend die folgenden Schritte:
a) Bestimmen des Niveaus (i) eines Anti-IL-17-Auto-Antikörpers und/oder (ii) eines Komplexes [IL-17/Anti-IL-17-Auto-Antikörper] in einer biologischen Probe des Individuums, bewertet durch das Verfahren gemäß Anspruch 1;
b) Auswählen eines Anti-IL-17-Auto-Antikörpers oder einer Produzenten-Zelle eines Anti-IL-17-Auto-Antikörpers ausgehend von einer biologischen Probe des Individuums; wobei der Auto-Antikörper zum Behandeln der Krankheit und/oder der Knochenzerstörung geeignet ist.

4. In-vitro-Verfahren zum Unterscheiden einer destruktiven rheumatoiden Polyarthritis (PR) von einer nicht-destruktiven PR bei einem Individuum, umfassend die folgenden Schritte:
a) Bestimmen (i) des Niveaus eines Anti-IL-17-Auto-Antikörpers und/oder (ii) des Niveaus eines Komplexes [IL-17/Anti-IL-17-Auto-Antikörper] in einer biologischen Probe des Individuums, wobei die biologische Probe aus Vollblut, Plasma und Serum ausgewählt ist, und
b) Vergleichen des in Schritt a) bestimmten Niveaus des Auto-Antikörpers und/oder des Komplexes mit einem Referenzwert, wobei der Referenzwert bestimmt wird (i) bei Individuen ohne eine Knochenzerstörung und/oder deren rheumatoide Polyarthritis mit der Produktion von Anti-IL-17-Auto-Antikörper und/oder Komplex [IL-17/Anti-IL-17-Auto-Antikörper] assoziiert ist, oder (ii) bei Individuen mit einer Knochenzerstörung und/oder bei Individuen, deren rheumatoide Polyarthritis nicht mit der Produktion von Anti-IL-17-Auto-Antikörper und/oder Komplex [IL-17/Anti-IL-17-Auto-Antikörper] assoziiert ist, wobei der Vergleich eine destruktive oder nichtdestruktive rheumatoide Polyarthritis (PR) bei dem Individuum anzeigt.

5. In-vitro-Verfahren gemäß einem der vorhergehenden Ansprüche, wobei der Schritt a) aus dem Bestimmen des Niveaus eines Anti-IL-17-Auto-Antikörpers in der biologischen Probe besteht.

6. In-vitro-Verfahren gemäß einem der vorhergehenden Ansprüche, wobei der Schritt a) aus dem Bestimmen des Niveaus eines Komplexes [IL-17/Anti-IL-17-Auto-Antikörper] in der biologischen Probe besteht.

7. In-vitro-Verfahren gemäß einem der Ansprüche 1-3, wobei die biologische Probe ausgewählt ist aus: Vollblut, Plasma, Serum, Synovialflüssigkeit, Zerebrospinalflüssigkeit, Pleuraflüssigkeit und Peritonealflüssigkeit.

8. In-vitro-Verfahren gemäß einem der vorhergehenden Ansprüche, wobei der Anti-IL-17-Auto-Antikörper ein Anti-IL-17A-Auto-Antikörper ist; und der Komplex [IL-17/Anti-IL-17-Auto-Antikörper] ein Komplex [IL-17A/Anti-IL-17A-Auto-Antikörper] ist.

9. In-vitro-Verfahren gemäß einem der vorhergehenden Ansprüche, wobei der Schritt a) in der Form eines ELISA-Tests durchgeführt wird.

10. In-vitro-Verfahren gemäß einem der vorhergehenden Ansprüche, ferner umfassend einen Schritt zur Bestimmung, in einer biologischen Probe des Individuums, des Niveaus eines Polypeptids, ausgewählt aus: den Zytokinen IL-17A, IL-17F, IL-25, IL-23, dem Transkriptionsfaktor RORγt und den Rezeptoren IL-17RA, IL-17RB, IL-17RC, den Auto-Antikörpern, die gegen IL-1α gerichtet sind, den Auto-Antikörpern, die gegen IL-8 gerichtet sind, und/oder den Auto-Antikörpern, die gegen Osteopontin gerichtet sind.

11. Isolierter Anti-IL17-Antikörper zur Verwendung bei der Behandlung oder Prävention (i) einer destruktiven rheumatoiden Polyarthritis oder (ii) einer rheumatoiden Polyarthritis bei einem Individuum, das keine Anti-IL-17-Auto-Antikörper produziert, oder einer mit einer der Gruppen (i) oder (ii) assoziierten Knochenzerstörung, wobei das Individuum durch das Verfahren gemäß einem der Ansprüche 4 bis 10 identifiziert wird.

## Claims

1. In vitro method for evaluating the prognosis of a chronic autoimmune or inflammatory disease in an individual, comprising the following steps:
a) determining (i) the level of an anti-IL-17 autoantibody and/or (ii) the level of an [IL-17/anti-IL-17 autoantibody] complex in a biological sample from said individual, and
b) comparing the level of autoantibody and/or of complex determined in step a) with a reference value, said comparison being indicative of the prognosis of a chronic autoimmune or inflammatory disease in said individual;
in which the chronic autoimmune or inflammatory disease is rheumatoid arthritis (RA).

2. In vitro method according to Claim **1,** for evaluating the prognosis of a chronic autoimmune or inflammatory disease in an individual for
(i) determining the risk of bone destruction in said individual suffering from said chronic autoimmune or inflammatory disease; and/or
(ii) determining the chances of response, of said individual suffering from said chronic autoimmune or inflammatory disease, to a treatment comprising the administration of an IL-17-inhibiting active ingredient;
and/or
(iii) determining the efficacy of a treatment or of a prevention of bone destruction in said individual suffering from said chronic autoimmune or inflammatory disease, said treatment or said prevention consisting of the administration of an IL-17-inhibiting active ingredient;
in which the comparison in step b) is indicative of said risk of bone destruction, of said chances of response to said treatment, and/or of the efficacy of said treatment or of said prevention of bone destruction.

3. In vitro method for selecting an antibody for treating (i) a chronic autoimmune or inflammatory disease, and/or (ii) bone destruction associated with said disease, in which the chronic autoimmune or inflammatory disease is rheumatoid arthritis (RA), comprising the following steps:
a) determining the level (i) of an anti-IL-17 autoantibody and/or (ii) of an [IL 17/anti-IL-17 autoantibody] complex in a biological sample from an individual evaluated by the method according to Claim 1;
b) selecting an anti-IL-17 autoantibody or a cell producing an anti-IL-17 autoantibody from a biological sample of said individual; said autoantibody being capable of treating said disease and/or said bone destruction.

4. In vitro method for distinguishing destructive rheumatoid arthritis (RA) from non-destructive RA in an individual, comprising the following steps:
a) determining (i) the level of an anti-IL-17 autoantibody and/or (ii) the level of an [IL-17/anti-IL-17 autoantibody] complex in a biological sample from said individual, the biological sample being chosen from whole blood, plasma and serum, and
b) comparing the level of autoantibody and/or of complex determined in step a) with a reference value, said reference value being determined (i) in individuals who do not have bone destruction and/or whose rheumatoid arthritis is associated with the production of anti-IL-17 autoantibody and/or of [IL-17/anti-IL-17 autoantibody] complex, or (ii) in individuals with bone destruction and/or in individuals whose rheumatoid arthritis is not associated with the production of anti-IL-17 autoantibody and/or of [IL-17/anti-IL-17 autoantibody] complex, said comparison being indicative of destructive or non-destructive rheumatoid arthritis (RA) in said individual.

5. In vitro method according to one of the preceding claims, in which step a) consists in determining the level of an anti-IL-17 autoantibody in said biological sample.

6. In vitro method according to one of the preceding claims, in which step a) consists in determining the level of an [IL-17/anti-IL-17 autoantibody] complex in said biological sample.

7. In vitro method according to one of Claims 1-3, in which the biological sample is chosen from: whole blood, plasma, serum, synovial fluid, cerebrospinal fluid, pleural fluid and peritoneal fluid.

8. In vitro method according to one of the preceding claims, in which the anti-IL-17 autoantibody is an anti-IL-17A autoantibody; and the [IL-17/anti-IL-17 autoantibody] complex is an [IL-17A/anti-IL-17A autoantibody] complex.

9. In vitro method according to one of the preceding claims, in which step a) is carried out in the form of an ELISA assay.

10. In vitro method according to one of the preceding claims, also comprising a step of determining, in a biological sample from said individual, the level of a polypeptide chosen from: the cytokines IL-17A, IL-17F, IL-25, IL-23, the transcription factor RORγt, and the IL-17RA, IL-17RB, IL-17RC receptors, autoantibodies directed against IL-1α, autoantibodies directed against IL-8 and/or autoantibodies directed against osteopontin.

11. Isolated anti-IL-17 antibody, for use in the treatment or prevention (i) of destructive rheumatoid arthritis or (ii) of rheumatoid arthritis in an individual not producing anti-IL-17 autoantibodies, or of bone destruction associated with either one of groups (i) or (ii), said individual being identified by the method according to one of Claims 4 to 10.
